# EUROPEAN PATENT SPECIFICATION

(11) **EP 1 593 685 B1**
(45) Date of publication and mention of the grant of the patent: **09.07.2008**
(21) Application number: 04252588.1
(22) Date of filing: 04.05.2004
(51) Int. Cl.: C07K 5/08, C07K 5/10, C07K 7/06, C12P 21/06, A23J 3/34, A23J 3/16, A61K 38/06, A61K 38/07, A61K 38/08, A61K 38/01

(54) **Peptide antioxidants from soy protein**
Peptid-Antioxidantien aus Sojaproteinen
Antioxidants peptidiques provenant de protéines de soja

(43) Date of publication of application: 09.11.2005
(73) Proprietor: KRAFT FOODS HOLDINGS, INC., Northfield, Illinois 60093 (US)
(72) Inventor: Gao, Song, Edison, New Jersey 08820 (US); Miller, Miranda, Arlington Heights, Illinois 60004 (US); Han, Xiao Quing, Naperville, Illinois 60540 (US)
(74) Representative: Smaggasgale, Gillian Helen

(56) References cited:
- EP-A- 0 657 520
- WO-A-03/097791
- SONNET, PASCAL ET AL: "First synthesis of segetalins B and G: two cyclopentapeptides with estrogen-like activity" TETRAHEDRON LETTERS , 44(16), 3293-3296 CODEN: TELEAY; ISSN: 0040-4039, 2003, XP004417090
- SAITO KOICHIRO ET AL: "Antioxidative properties of tripeptide libraries prepared by the combinatorial chemistry." JOURNAL OF AGRICULTURAL AND FOOD CHEMISTRY, vol. 51, no. 12, 4 June 2003 (2003-06-04), pages 3668-3674, XP002298641 ISSN: 0021-8561
- HERRAIZ TOMAS ET AL: "Endogenous and dietary indoles: A class of antioxidants and radical scavengers in the ABTS assay." FREE RADICAL RESEARCH, vol. 38, no. 3, March 2004 (2004-03), pages 323-331, XP009037286 ISSN: 1071-5762
- DATABASE CHEMABS [Online] CHEMICAL ABSTRACTS SERVICE, COLUMBUS, OHIO, US; 12 May 1984 (1984-05-12), HIYOSHI, AKIRA ET AL: "Preventing oxidation of oils and fats of food" XP002298503 retrieved from STN Database accession no. 1974:46644 & JP 480 149 42B B4 (RESEARCH AND DEVELOPMENT CENTER, JAPAN DEFENSE AGENCY) 11 May 1973 (1973-05-11)
- CHEN H-M ET AL: "STRUCTURAL ANALYSIS OF ANTIOXIDATIVE PEPTIDES FROM SOYBEAN BETA-CONGLYCININ" JOURNAL OF AGRICULTURAL AND FOOD CHEMISTRY, AMERICAN CHEMICAL SOCIETY. WASHINGTON, US, vol. 43, no. 3, 1995, pages 574-578, XP001027076 ISSN: 0021-8561
- CANDELARIO-MARTINEZ A ET AL: "SCP-related peptides from bivalve mollusks: Identification, tissue distribution, and actions" BIOLOGICAL BULLETIN (WOODS HOLE), vol. 185, no. 3, 1993, pages 428-439, XP002298642 ISSN: 0006-3185

### SEQUENCE LISTING

<110> Kraft Foods Holdings, Inc.
<120> Peptide Antioxidants from Soy Protein
<130> P504815EP
<140> Not yet known
   <141> 2004-05-04
<160> 6
<170> PatentIn Ver. 2.1
<210> 1
   <211> 4
   <212> PRT
   <213> Artificial Sequence
<220>
   <223> Description of Artificial Sequence:Glycine max
<400> 1
   Gly Val Ala Trp
    1
<210> 2
   <211> 5
   <212> PRT
   <213> Artificial Sequence
<220>
   <223> Description of Artificial Sequence:Glycine max
<400> 2
   Gly Val Ala Trp Trp
    1               5
<210> 3
   <211> 6
   <212> PRT
   <213> Artificial Sequence
<220>
   <223> Description of Artificial Sequence:Glycine max
<400> 3
   Gly Val Ala Trp Trp Met
    1               5
<210> 4
   <211> 7
   <212> PRT
   <213> Artificial Sequence
<220>
   <223> Description of Artificial Sequence:Glycine max
<400> 4
   Gly Val Ala Trp Trp Met Tyr
    1               5
<210> 5
   <211> 4
   <212> PRT
   <213> Artificial Sequence
<220>
   <223> Description of Artificial Sequence:Glycine max
<400> 5
   Trp Trp Met Tyr
    1
<210> 6
   <211> 5
   <212> PRT
   <213> Artificial Sequence
<220>
   <223> Description of Artificial Sequence:Glycine max
<400> 6
   Ser Trp Trp Pro Phe
    1               5

## Description

### Field of the Invention

The present invention is directed to antioxidant peptides that result from the proteolytic digestion of soy protein. The peptides may be used as additives in pharmaceutical or dietary compositions to protect compounds from oxidative degradation. Alternatively, the peptides may themselves be used as either therapeutic agents or dietary supplements.

### Background of the Invention

Soy protein has been reported to have a number of important health-promoting effects, including an ability to reduce the risk of cardiovascular disease and cancer *(*Potter, Nutr. Rev. 56:231-235 (1998); Greeves, J. Nutr. 130:820-826 (2000); J. Nutr. 125:698S-708S (1995); J. Nutr. 125:7335 S-743S (1995)). A possible explanation for these effects is suggested by the fact that peptides generated from soy using proteases have been shown to have antioxidant activity (Chen et al. J. Agric. Food Chem. 46:49-53 (1998); Chen et al. J. Agric. Food Chem. 43:,574-578 (1995);
Suetsuna, J. Jpn. Soc. Nutr. Food Sci. 52:225-228 (1999); See also Zhang, et al. Ann. NY Acad. Sci. 864: 640-645 (1998)). By scavenging free radicals and oxidative species generated during the course of *in vivo* reactions, the peptides may help protect against pathogenic processes involving enzyme inactivation, DNA mutation and protein denaturation (Szweda, et al. J. Biol. Chem. 268:3342 (1993); Reiss et al. Biochem. Biophys. Res. Commun. 48:921 (1972)).

In addition to having potential clinical use in reducing the risk of patients developing heart disease (Perticone, et al., Diabetes 50):159-165 (2001); Krinsky, Annu. Rev. Nutr. 13:561 (1993)) and cancer (Melov, et al., Science 289:1567-1569 (2000)), antioxidant peptides may be used to prevent oxidative damage in pharmaceutical and food products. In food products, the peptides can be used to prevent the oxidation of oils and fats, which is associated with loss of nutritious value and causing rancidity. In pharmaceutical products, they should help to maintain the structural integrity of drugs exposed to destructive oxygen radicals.

WO 03/097791 provides a method of identifying alkaline-alpha-galactosidases. Also provided are novel polynucleotide sequences encoding polypeptides having an alkaline-alpha-galactosidase activity, oligonucleotides and oligonucleotide analogs derived from the polynucleotide sequences, peptides and peptide analogues, antibodies recognizing same and methods of using same. However, there is no mention in WO 03/097791 that the peptides disclosed therein display antioxidant properties.

Sonnet et al, Tetrahedron Letters 44 (2003) p. 3293-3296 "First synthesis of segetalins B and G: two cyclopentapeptides with estrogen-like activity" discloses the synthesis of two segetalins B and G. The corresponding linear peptides were synthesised using standard automated continuous-flow SPPS methods. Ring closure positions were investigated for segetalin B. The best ring closure activity was found between Val and Gly. There is no mention in that journal article that any of the peptides disclosed therein display antioxidant properties.

Saito et al, Journal of Agricultural and Food Chemistry 2003, 51, p 3668-3674 discloses two series of combinatorial tripeptide libraries, based on an antioxidative peptide isolated from a soybean protein hydrolysate. One was a library of 108 peptides containing either His or Tyr residues. Another was a library of 114 peptides related to Pro-His-His, which had been identified as an active core of the antioxidative peptide. The antioxidative properties of these libraries were examined by several methods, such as the antioxidative activity against the peroxidation of linoleic acid, the reducing activity, the radical scavenging activity, and the peroxynitrite scavenging activity. Two Tyr-containg tripeptides showed higher activities than those of two His-containing tripeptides in the peroxidation of linoleic acid. Tyr-His-Tyr showed a strong synergistic effects with phenolic antioxidants. However, the tripeptide had only marginal reducing activity and a moderate peroxynitrite scavenging activity. Cysteine-containing tripeptides showed the strong peroxynitrite scavenging activity. Change of either the N-terminus or C-terminus of Pro-His-His to other amino acid residues did not significantly alter their antioxidative activity. Tripeptides containing Trp or Tyr residues at the C-terminus had strong radical scavenging activities, but very weak peroxynitrite scavenging activity. The present results allow us to understand why protein digests have such a variety of antioxidative properties.

Herraiz et al, Free Radical Research (March 2004) Volume 38 Number 3, pages 323-31 discloses a series of experiments in which a total of twenty-nine indoles and analogs were examined for their properties as antioxidants and radical scavengers against 2,2'-Azino-bis(3-ethylbenzthiazoline-6-sulfonic acid) ABTS radical cation. With only a few exceptions, indoles reacted nonspecifically and quenched this radical at physiological pH affording ABTS. Indoleamines like tryptamine, serotonin and methoxytryptamine, neurohormones (melatonin), phytohormones (indoleacetic acid and indolepropionic acid), indoleamino acids like L-tryptophan and derivatives (N-acetyltryptophan, L-abrine, tryptophan ethyl ester), indolealcohols (tryptophol and indole-3-carbinol), short peptides containing tryptophan, and tetrahydro-beta-carboline (pyridoindole) alkaloids like the pineal gland compound pinoline, acted as radical scavengers and antioxidants in an ABTS assay-measuring total antioxidant activity. Their trolox equivalent antioxidant capacity (TEAC) values ranged from 0.66 to 3.9 mM, usually higher than that for Trolox and ascorbic acid (1 mM). The highest antioxidant values were determined for melatonin, 5-hydroxytryptophan, trp-trp and 5-methoxytryptamine. Active indole compounds were consumed during the reaction with ABTS and some tetrahydropyrido indoles (e.g. harmaline and 1-methyl-1,2,3,4-tetrahydro-β-carboline-3-carboxylic acid ethyl ester) afforded the corresponding fully aromatic β-carbolines (pyridoindoles), that did not scavenge ABTS. Radical scavenger activity of indoles against ABTS was higher at physiological pH than at low pH. These results point out to structural compounds with an indole moiety as a class of radical scavengers and antioxidants. This activity could be of biological significance given the physiological concentrations and body distribution of some indoles.

EP0657520 provides a safe antioxidant applicable to foods, medicines, quasi-drugs, and other products. The antioxidant contains an active ingredient comprising an antioxidizing substance alone or a mixture thereof with an oxidation inhibitor, said antioxidizing substance being selected from the group consisting of decomposition products of lactoferrins, peptides isolated from the above decomposition products, synthetic peptides each having the same amino acid sequence as that of any of the synthetic peptides, and arbitrary mixtures of these compounds. This antioxidant has an excellent effect of inhibiting the oxidation of various lipids, is extremely safe for foods and drugs, and exhibits an antioxidant effect even in a small amount, so that it scarcely affects the taste, etc., of food or the like.

JP 48-014942 discloses a protein hydrolyzate which may be added to foods to retard the oxidation of oils and fats in those foods. The effectiveness of the hydrolyzate was demonstrated by adding 3-5% of the hydrolyzate to a 20% alcohol solution of linoleic acid which resulted in a significant delay in the production of peroxide. The hydrolyzate was formed by mixing 0.3g of protease with 100g of soybean protein in 1.2 litres of water. The mixture was incubated at 45°C for 24 hours. The resulting hydrolyzate, after drying, contained 1.3% amino acids and 84.2% peptides.

### Summary of the Invention

The present invention is based upon the isolation and characterization of peptides derived from soy protein that have a high degree of antioxidant activity. These peptides may be included in pharmaceutical or dietary compositions for the purpose of scavenging free radicals and preventing the oxidative decomposition of compounds.

Examples of such peptides consist of any one of the following amino acid sequences: GVAW (SEQ ID NO:1); GVAWW (SEQ ID NO:2); GVAWWM (SEQ ID NO:3); GVAWWMY (SEQ ID NO:4); GVAWWM*; and GVAWWM*Y; where the symbol M* refers to methionine sulfoxide and may also be abbreviated as either Met(O) or MetS(O). All of these sequences are shown as beginning at the N terminus (on the left) and ending at the C terminus (on the right). The peptides only contain the 1-amino acids that are commonly recognized in the art as being present in the mammalian or plant proteins.

Thus according to the present invention there is provided a method of reducing the oxidative degradation of fats or oils in a composition, comprising adding to said composition an effective amount of an isolated protein consisting of any of the following sequences:
GVAW;
GVAWW;
GVAWWM;
GVAWWMY;
GVAWWM*; and
GVAWWM*Y.

Peptides having the amino acid sequences represented by SEQ ID NO:1 - SEQ ID NO:4, as well as the methionine sulfoxide containing peptides, all appear to be the result of the proteolytic digestion of the G1, G2, or G3 subunit of glycinin (the 11S fraction of soy protein isolate). The remaining peptides appear to result from the digestion of the G4 precursor of glycinin, the G5 precursor of glycinin, the cytochrome C oxidase polypeptide II precursor, or the 62 K sucrose-binding protein precursor, all of which have sequences that are known in the art. The peptides can be obtained by processing soy protein isolate, soy protein concentrate and soy flour using the procedures described herein or, alternatively, they can be synthesized using solid or liquid phase methods that are routine in the art of peptide chemistry.

The term "isolated peptide" as used herein refers to a peptide that has been separated from other accompanying biological components and should typically comprise at least 85% of a sample, with greater percentages being preferred. The purity of preparations can be assessed using standard procedures such as electrophoresis, chromatography and analytical centrifugation.

The term "consisting essentially of" includes peptides having the exact sequences shown as well as peptides that do not differ substantially from those shown either in terms of structure or function. It is well recognized that conservative amino acid substitutions, *e.g.*, substituting one acidic, basic or neutral amino acid for another, can often be made without affecting the biological activity of a peptide or protein. Similarly, it is often possible to add one or more amino acids to the N or C terminus of peptides without substantially reducing activity. Minor variations in sequence of this nature are encompassed by the term "consisting essentially of" provided, that these changes do not substantially reduce antioxidant activity. For the purposes of the present invention, modifications in sequence are limited to the substitution of no more than one amino acid per peptide and/or the addition of no more than one or two amino acids to the N and/or C terminus of a peptide. Any structural change that substantially reduces antioxidant activity (*e.g.*, by 15% or more as determined using one of the assays described herein or using another assay accepted in the art) results in a peptide that is not a part of the invention.

The antioxidant peptides used in the method of the present invention may be used in pharmaceutical or dietary compositions. Pharmaceutical compositions may be formulated using standard procedures in the art and may be designed for any route of administration. In some pharmaceutical compositions, peptides will be added for the purpose of preventing the oxidative destruction of active therapeutic agents and should be present in an amount sufficient to effectively scavenge free radicals. For example, a peptide may be present in liquid compositions at between 0.1 µg/ml and 1.0 mg/ml and, in solid dosage forms, at between .000 1 % and 10% of the total weight of the composition.

In other cases, the peptide itself may constitute a primary active ingredient in pharmaceutical compositions designed to treat pathological conditions associated with excessive oxidation. In these cases, a peptide will typically constitute a much higher percentage of a dosage form. For example, it may constitute 1-100% of a solid dosage form or be present in liquid dosage forms at a concentration ranging from 0.1 mg/ml to 10 mg/ml. The peptide may constitute the sole active ingredient or be combined with other therapeutic agents, *e.g.*, other antioxidants. Among the conditions that may be treated using pharmaceutical compositions containing the antioxidant peptides are hypertensive conditions, inflammatory conditions, allergic conditions, and autoimmune conditions. Specific examples include arthritis, diabetes, neurodegenerative diseases, cancer and multiple sclerosis. The use of antioxidants has also recently proven useful in preventing tissue damage after traumatic injury (*e.g*., to the spinal column), after a stroke or after a heart attack. In general, patients should be able to tolerate very high levels of antioxidants (*e.g*., between 50 mg and 10 g per day) and exact dosages can be determined using methods well established among clinicians.

The peptides of the present invention may also serve as additives in dietary compositions in two different ways. First, they may be added to a food composition for the purpose of preventing the oxidation of oils or fats. Under these circumstances, the peptides may be present in a very wide range of concentrations. For example, it may constitute from about 0.0001 % to about 10.0% of the total weight of the food composition. Examples of oils and fats that may be protected using the peptides include frying oil, vegetable oil, corn oil, olive oil, soybean oil, palm oil, safflower oil, olive oil, sunflower oil, cotton seed oil, natural fats, lards and synthetic fats. These and similar compounds may be present in a very wide range of food products including meats, dairy products and processed foods.

Alternatively, the peptides may be present in a supplement designed to augment a person's diet. In this case, the peptide may constitute a much higher percentage of the total weight of the product (*e.g.*, from .01% - 100%) and may be combined with other supplements such as vitamins and minerals. The dietary supplements containing the peptide can be designed for over the counter consumption, in which case the supplements will typically be in the form of a tablet, capsule or solution. Alternatively, the supplements can be designed for administration in a hospital setting, in which case they may be present as part of a liquid composition for parenteral administration. In general, people should be able to safely take anywhere from 50 mg up to 10 or more grams of a peptide supplement per day.

The peptides used in the method of the present invention are made by digesting soy protein isolate (available commercially, *e.g.*, from Protein Technology International, St. Louis, MO, or using procedures well established in the art) with a protease to produce an aqueous hydrolysate solution. Proteases that may be used for digestion include chymosin, papain, neutrase, promod, and flavozyme and, preferably, either alcalase or corolase. The protease may come from bacterial, plant or fungal sources. The aqueous hydrolysate solution is extracted with an organic solvent, preferably acetone, and the organic phase is selected for further processing. After extraction, solvent may be removed under reduced pressure to concentrate the peptide and the preparation is then fractionated by reverse phase high pressure liquid chromatography (HPLC). Fractions collected during this process may be assayed for antioxidant activity using methods such as those described in the Examples section herein. The identification of fractions containing the peptide may be based upon the presence of antioxidant activity and upon retention time during chromatography. The identity of the peptide within fractions can be confirmed by sequence analysis.

### Brief Description of the Drawings

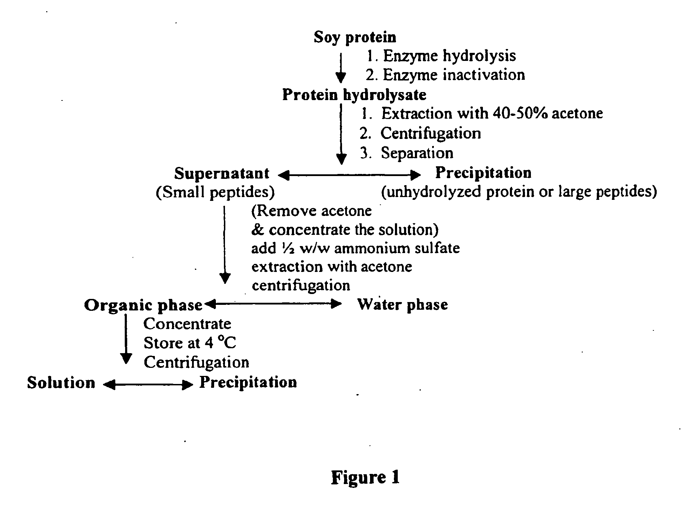

Figure 1: Figure 1 is a diagrammatic representation showing a method for processing soy protein, for example soy protein isolate, to partially purify antioxidant proteins. The final solution and precipitant shown in the figure are further processed by reversed phase HPLC.

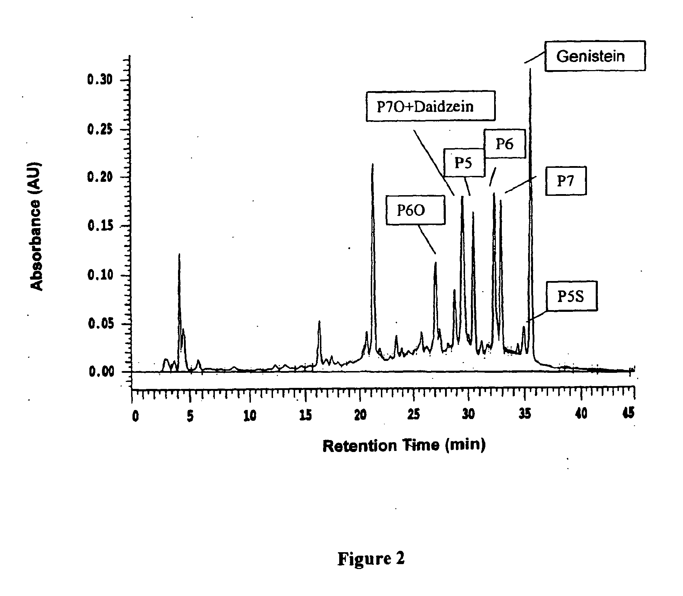

Figure 2: Reverse phase HPLC spectrum of the antioxidative peptides isolated from soy protein hydrolysate. Soy protein, for example soy protein isolate, was digested with either alcalase or corolase and processed as described in the Examples section. The final step in the purification involves reverse phase HPLC and a chromatogram obtained from this step is shown in Figure 2. The various peaks shown in the figure are as follows: P5: GVAWW (SEQ ID NO:2); P5S: FWWPF (SEC ID NO:6); P6: GVAWWM (SEC ID NO:3); P6O: GVAWWM*; P7: GVAWWMY(SEC ID NO:4); and P7O: GVAWWM*Y.

### Detailed Description of the Invention

The present invention is based upon the identification of antioxidant peptides produced by the enzymatic hydrolysis of soy protein. Several of the peptides were found to be fragments of G1, G2, and G3 subunits of glycinin, the most abundant soybean protein. Spectroscopic analysis of the purified peptides indicated their weights as being 431, 617, 748, 764, 911 and 927 Daltons. Sequencing experiments revealed sequences of: GVAW (SEQ ID NO:1); GVAWW (SEQ ID NO:2); GVAWWM (SEQ ID NO:3); GVAWWM*; GVAWWMY(SEQ ID NO:4); and GVAVAVM*Y. Additional soy-derived peptides found to have antioxidant activity which are mentioned here for exemplary purposes only and do not fall within the scope of the claim were: WWMY (SEQ ID NO:5); SWWPF (SEQ ID NO:6); and WTY.

Preferably, the antioxidant peptides are produced from soy protein isolate using the procedures described below in the Examples section. However, alternative procedures may also be used. For example, the peptides may be chemically synthesized using liquid or solid phase procedures that are routine in the art (see *e.g.,* Merrifield, J. Am. Chem. Soc. 85:7129(1963); Meienhofer, et al., J. Pept. Prot. Res. 13:35(1979); and Fields et al. Pept. Res. 4 95(1991)). Alternatively, the peptides can be produced recombinantly by constructing a nucleic acid sequence coding for the peptide, incorporating this into an expression vector in which the coding sequence is placed under the control of a promoter, transforming an appropriate host cell with the vector, and then incubating the transformed host under conditions by which peptide is synthesized. The peptide may be purified from either the host cell or from the medium in which the cell is grown.

The antioxidant peptides may be used in cosmetic, pharmaceutical or dietary compositions. In either case, the peptide may be present in a relatively low amount (*e.g.* .0001% -10% of the total weight of the composition and preferably from .001% to 1 %) for the purpose of scavenging free radicals and preventing other compounds from undergoing oxidative degradation. Alternatively, peptide may itself be an active ingredient for the treatment of a disease or condition associated with excessive oxidation or it may serve as a dietary supplement. In these instances, the peptide may constitute from 0.01 % to 100% of a solid composition or be present in a liquid composition at between 0.1 mg/ml and 10 mg/ml.

As pharmaceutical agents, the antioxidant peptides are compatible with any dosage form or route of administration. Specifically, the peptides may be administered intranasally, parenterally or transdermally. Dosage forms may include tablets, capsules, caplets, lozenges, parenterals, liquids, powders, and formulations designed for implantation or administration to the surface of the skin. It is expected that oral dosage forms will be the most convenient in most instances but parenterals may also be useful in a hospitable setting. For example, peptides may be included as part of an intravenous drip administered to a patient after traumatic injury, stroke, or heart attack. All dosage forms may be prepared using methods that are standard in the art (see *e.g.*, Remington's Pharmaceutical Sciences, 16th ed., A. Oslo ed., Easton, PA(1980)).

Active peptides may be used in conjunction with any of the vehicles and excipients commonly employed in pharmaceutical compositions, *e.g.,* talc, gum arabic, lactose, starch, magnesium sterate, cocoa butter, aqueous or non-aqueous solvents, oils, paraffin derivatives, glycols, etc. Coloring or flavoring agents may also be added to preparations designed for oral administration. Solutions can be prepared using water or physiologically compatible organic solvents such as ethanol, 1-2 propylene glycol, polyglycols, dimethyl sulfoxide, fatty alcohols, triglycerides, partial esters of glycerin, and the like. Parenteral compositions containing peptides may be prepared using conventional techniques and include sterile isotonic saline, water, 1,3-butanediol, ethanol, 1,2-propylene glycol, polyglycols mixed with water, Ringer's solution, etc.

The daily dosage administered to a patient may be provided in either a single or multiple regimen depending on a variety of clinical circumstances. Optimal dosages will be determined by methods well known in the art and will be influenced by clinical factors such as the age and state of the patient and the disease or disorder being treated. In general, it expected that a suitable dosage range for intravenous administration will be between 10 and 500 micrograms of peptide per kilogram patient body weight. When administered orally, dosages will typically be significantly higher and will, usually, be in a range of between 50 mg per day and 10 grams per day. When used as part of a dietary supplement, peptide will usually be administered orally in the form of a tablet, capsule or liquid. The peptides may be combined with other dietary supplements such as vitamins and minerals.

The antioxidant peptides used in the method of the present invention may be produced from soy protein, preferably soy protein isolate. The first step of this process involves digesting the soy protein with a protease under conditions sufficient to result in the generation of peptide. Although many different proteases may be used, alcalase and corolase have been found to be particularly effective. The exact conditions for digestion may vary over a wide range but in a preferred embodiment, digestion is carried out at 50° C for a period of about three hours. After inactivation of the enzyme, the digestion mixture is extracted with organic solvent. For example, extraction may take place using approximately an equal volume of acetone. The supernatant of the extraction, *i.e.*, the organic phase is recovered and solvent is removed under reduced pressure.

After recovering peptide from the extraction, reverse phase high pressure liquid chromatography may be performed using, for example, a C 18 column. A variety of solvent systems may be used for eluting the column but a gradient from 5-60% acetonitrile in phosphate buffer at pH6.8 has been found to be effective. Fractions collected from the column may be tested for antioxidant activity to identify peaks that are of interest. Finally, specific peptides may be identified by carrying out sequencing experiments.

### Examples

### A. Antioxidant Activity Recovered After Enzyme Treatment

Three flat bottom glass bottles containing 100 ml of 5% PTI XT 34 soy protein isolate were incubated in a 50°C water bath with 0.4 ml alcalase. The incubation was stopped at 1 hour, 2 hours and 4 hours by putting the bottle in an 80-85°C water bath for ten minutes. Control preparations were obtained by first inactivating the alcalase enzyme at 80-85° C for at least 10 minutes and then adding the inactivated enzyme preparation to 100 ml of a 5% SPI solution. After centrifugation in an Eppendorf microcentrifuge, supernatants were diluted 5-fold. Free amino groups were measured as leucine equivalents as an estimate of peptide released during hydrolysis and compositions were diluted 10-fold and used to perform ABTS radical assays (described more fully below). The results indicated that antioxidant activity increased as hydrolysis proceeded and peptides were released.

### B. Screen of Soy Protein Hydrolysis by Different Enzymes

A 14% total solid soy protein suspension was prepared by mixing 700 g of soy protein isolate (SuproXT34) with 4300 g of hot water (approximately 60°C). 750 g of the resulting suspension was aliquoted and incubated with enzyme under the conditions shown in Table 1.

**Table 1**

| **Sample** | **Enzyme** | **Reaction Conditions** |
|---|---|---|
| 1. | N-22 (1ml)/chymosin(1ml) | 45° C 220 min. |
| 2. | N-22 (1ml)/papain (1ml) | 50° C 220 min. |
| 3. | N-22 (1ml)/corolase (1ml) | 50° C 220 min. |
| 4. | 446 (1ml)/chymosin (1ml) | 45° C 220 min. |
| 5. | 446 (1ml)/papain (1ml) | 50° C 200 min. |
| 6. | 446 (1ml)/corolase (1ml) | 50° C 200 min. |
| 7. | alcalase (1ml) | 50° C 240 min. |
| 8. | neutrase (1ml) | 50° C 240 min. |
| 9. | promod 24L (1ml) | 50° C 220 min. |
| 10. | flavozyme (1ml) | 50° C 220 min. |

| | | |
|---|---|---|
| ■ N-22: Protease NS 37022 (Novo Nordisk); ■ alcalase (Novo Nordisk); ■ 446: Glutamyl Endopeptidase (SP 446) (Novo Nordisk); ■ Corolase PN-L (Creanova Inc); ■ Papain 6000 (Valley Research Inc, Hammond IN); ■ Promod 24L (Biocatalysts Ltd., Mid Glamorgan, UK); ■ Flavozyme (Novo Nordisk); ■ Neutrase: Neutral Bacterial Protease (Medipharm AB, Kagerod Sweden). | | |

One volume of acetone was added to precipitate out unhydrolyzed, intact proteins and/or large polypeptides. The residue was separated by centrifugation and washed with about one volume of 50% acetone. Organic solvent was then removed using a rotary evaporator at 40-60°C. The sample at this step was designated as SP 1-10. Half the weight of SP1-10 of ammonium sulfate was then added to the hydrophobic peptides and the mixture was centrifuged. Phase I, which contained acetone was the upper "oily" phase, and phase II was the lower aqueous phase. The aqueous phase was further extracted with acetone and combined with phase I. The extract was concentrated and designated as SE1-10. The concentration of peptides from the paired protein hydrolysate (SP to SE) was then determined.

The concentration of peptides in samples was determined by the trinitrobenzene sulfonic acid (TNBS) method using leucine as a standard (Adler-Nissen, J. Agric. Food Chem. 27: 1256 (1979)). Equal volumes of sample in about 0.2 M phosphate buffer, pH 8.2, and a 0.1 % TNB solution were combined and incubated at 50°C for one hour. A volume of 0.1 N HCl equal to the total volume of the combined sample and TNB solution was added to stop the reaction. The sample was then cooled to room temperature. Absorbance at 415 nm was read after 30 minutes.

Table 2 shows the concentration of peptides in the samples prepared using different proteases. Peptide levels change due to differences in enzyme specificities, activities, product profiles and reaction conditions. The results for SP and SE are shown below because these samples were particularly rich in peptide antioxidant activity. Antioxidant activity of the samples evaluated by an ABTS radical assay is also summarized in Table 2.

**Table 2**

| Sample | Leucine Equivalent (mM) SP | Leucine Equivalent (mM) SE | Trolox Equivalent SP | Trolox Equivalent SE |
|---|---|---|---|---|
| 1 | 32.9 | 54 | 0.46 | 0.40 |
| 2 | 55 | 95 | 0.37 | 0.54 |
| 3 | 78.9 | 110 | 0.39 | 0.61 |
| 4 | 40.5 | 69 | 0.56 | 0.50 |
| 5 | 69.4 | 109 | 0.40 | 0.56 |
| 6 | 99.3 | 109 | 0.48 | 0.57 |
| 7 | 132 | 135 | 0.56 | 0.60 |
| 8 | 73.9 | 136 | 0.42 | 0.58 |
| 9 | 63.8 | 137 | 0.38 | 0.57 |
| 10 | 96.4 | 146 | 0.41 | 0.51 |

### C. Soy Protein Hydrolysis and Peptide Extraction

The procedure for hydrolyzing soy protein and extracting peptide is shown schematically in Figure 1. The process proceeds as follows:
1. 18 g soy protein isolate is mixed with 162 ml water to obtain a 10% mixture.
2. 1 ml of enzyme is added and the preparation is incubated in a 50° C water bath for three hours.
3. Enzyme is inactivated at 80-85° C for ten minutes.
4. After cooling to room temperature, 160 ml of the treated mixture is mixed with 106 ml acetone.
5. The preparation is centrifuged at 15,000-22,000 g for thirty minutes to obtain the supernatant.
6. The pellet from the centrifugation step is mixed with 200 ml of 50% acetone.
7. Centrifugation is repeated and the supernatants are combined. If a precipitate appears, the preparation is centrifuged again the supernatant recovered.
8. Acetone is removed under reduced pressure under a rotary evaporator.
9. If a precipitate appears, the preparation is centrifuged.
10. Half the weight amount of ammonium sulfate is dissolved into the supernatant.
11. The solution is extracted with 1/3-1/2 volumes of acetone two to three times.
12. The organic phase is concentrated and the sample is then stored in a refrigerator before analysis. If a precipitate forms during storage, both the supernatant and precipitant are used for antioxidant peptide isolation.

### D. Peptide Isolation by HPLC

### Samples

1. Soy Protein Isolate (ADBM-891 treated with alcalase)
2. Soy Protein Isolate (ADBM-891 treated with corolase-PNL)

### Supernatant

In order to prepare samples for HPLC, the supernatant is dried by nitrogen flow and the residue is mixed with mobile phase A (5% acetonitrile (ACN) in 10 mM sodium phosphate buffer, pH 6.8) and centrifuged to remove undissolved material before analysis and isolation. The prepared sample is injected onto a HPLC system.

Reverse phase HPLC is performed using a Lachrom Purospher RP-18 column, a Hitachi L-7100 pump, a L-7200 auto sampler, a L-7455 diode array detector and a D-7000 HPLC system manager software (Hitachi, Instruments, Inc. San Jose, CA). Two mobile phase (eluents) are used in the HPLC separation:
A: 5% ACN in 10 mM sodium phosphate buffer, pH 6.8;
B: 60% ACN, 40% 10 mM sodium phosphate buffer, pH 6.8

The flow rate of columns is set to 0.6 ml/min and ultraviolet absorbance is set at 275 nm. The gradient elution program is set as follows:
0-2 min, 90% A mobile phase/10% B mobile phase;
2-40 min, from 90% A mobile phase/10% B mobile phase to 100% B mobile phase.

Fractions with antioxidant activity are collected and subjected to further purification. This is carried out using the mobile phases:
A: 0.1 % trifloroacetic acid (TFA), 5% ACN
B: 0.05% TFA, 80% ACN.

The flow rate is set to 0.6 ml/min, 0.45 ml/min, or 0.4 ml/min and ultraviolet absorbance is set at 275 nm. The gradient elution program is set as follows:
0-2 min, 90% A mobile phase/10% B mobile phase;
2-25 min from 90% A mobile phase/10% B mobile phase to 60% A mobile phase/40% B mobile phase;
25-40 min, from 60% A mobile phase/40% B mobile phase to 10% A mobile phase/90% B mobile phase.

Fractions with antioxidant activity are collected and subject to further purification. In this case, the same mobile phases are used. The flow rate is set at 0.4 ml/min and ultraviolet absorbance is set at 275 nm. The gradient elution program is set as follows:
0.2 min 70% A mobile phase 30% B mobile phase;
2-40 min, from 70% A mobile phase/30% B mobile phase to 100% B mobile phase.

### Precipitant

The precipitant from the above sample or nitrogen blow-dry sample is dissolved in a solution of 80% acetonitrile, 0.05% trifloroacetic acid. The same basic HPLC system is used. The mobile phases are:

### A: 0.1% TFA, 5% ACN

### B: 0.05% TFA, 80% ACN

The flow rate is set at 0.6 ml/min and ultraviolet absorbance is set at 275 nm. The gradient elution program is set as follows:
0 - 2 min, 90% A mobile phase/10% B mobile phase
2 - 25 min from 90% A mobile phase/10% B mobile phase to 60% A mobile phase/40% B mobile phase.
25 - 40 min from 60% A mobile phase/40% B mobile phase to 10% A mobile phase/90% B mobile phase.

A typical chromatogram is shown as Figure 2.

### E. Peptide Sequence Determination

The sequences of peptides isolated by HPLC were determined by HPLC-MS. Gradient mobile phase compositions containing 0.05% TFA in water and 0.05% TFA in acetonitrile were selected for separation of peptides using a Waters Alliance 2690 HPLC (Waters Corporation Milford, MA). The peptides were separated using a reverse-phase 2.1 x 150 mm, 3.5 µm particle size Symmetry C-18 column (Waters Corporation Milford, MA) held at 40°C. The flow rate was 200 µL/min which was split 80:120 between Waters 2487 Dual UV Wavelength Absorbance Detector and Finnegan MAT TSQ 700 mass spectrometer (ThermoQuest, San Jose, CA). The Waters 2487 Dual UV Wavelength Absorbance Detector was set to acquire UV data at 214 and 280 nm. Peptide mass analyses were performed on the TSQ700 utilizing the Finnigan API 1 ion source interface in the positive electrospray ionization mode with a heated capillary at a temperature of 260°C. A retention-time-dependence automated HPLC/MSMS method was used to acquire product-ion tandem mass spectra using a computer program written in the TSQ700's instrument control line (ICL). All tandem mass spectrum were acquired with the collision cell filled with argon to a pressure of between 2 and 3.5 mTorr and collision energies between -10 to -44 eV. Tandem mass spectrometry data was analyzed *de novo* using the computer program SEQUEST (licensed from Therma Quest and operated on a Windows NT 4.0 Xcalibur workstation) to identify peptide sequences in a downloaded soybean database under "glycine" for *Glycine max* from the National Center for Biotechnology Information. The sequences are shown in Table 3. Those sequences not used in the method of the present invention are included for exemplary purposes only.

**Table 3**

| Sequence Determined | SEQ ID NO | Mol. Wt. | Source |
|---|---|---|---|
| GVAW | SEQ ID NO:1 | 431 | 11S: G1, G2, & G3 subunit |
| GVAWW | SEQ ID NO:2 | 617 | 11S: G1, G2, & G3 subunit |
| GVAWWM | SEQ ID NO:3 | 748 | 11S: G1, G2, & G3 subunit |
| GVAWWM* | - | 764 | 11S: G1, G2, & G3 subunit |
| GVAWWMY | SEQ ID NO:4 | 911 | 11S: G1, G2, & G3 subunit |
| GVAWWM*Y | - | 927 | 11S: G1, G2, & G3 subunit |
| WWMY | SEQ ID NO:5 | 684 | 11S: G1, G2, & G3 subunit |
| SWWPF | SEQ ID NO:6 | 721 | Sucrose binding protein |
| WTY | - | 468 | 11S: G4, G5, cytochrome C oxidase polypeptide II |

### F. Peptides of the Invention May Be Synthesized

Methods for synthesizing peptides are well known to those skilled in the art of amino chemistry, food chemistry, immunology and/or protein chemistry. For example, peptides are synthesized using solid phase Fmoc chemistry according to the principles initially described by Merrifield (J. Am. Chem. Soc. 85; 7129 (1963)) as modified subsequently by Meienhofer et al. (J. Peptide Prot. Res. 13; 35 (1979)) and Fields, et al., Peptide Res. 4; 95 (1991)). Typically, such synthesis is carried out on automated peptide synthesizers, such as those available from Applied Biosystems (Foster City, CA). Once synthesized, sequences are typically verified using an automated peptide sequencer such as a Porton model 2090 (Beckman Instruments Inc., Mountain View, CA).

### G. Antioxidant Assay Methods

### ABTS Radical Assay

This assay measures the total reducing power of peptides with respect to a preformed ABTS radical according to the methods of Pellegrini, et al. (Meth. Enzymol. 299:379 1999)). In the assay, 0.175 ml of 20 mM phosphate buffer (PB, pH 6.8) and 1.0 ml ABTS radical (0.837 in A734 nm) solution are combined in an Ep-tube. 0.025 ml of sample is added to the solution. The mixture is agitated and absorbance at 734 nm is measured after ten minutes. Sample solvent is used as a blank where appropriate. The working solution of ABTS radical is prepared by adding one volume of a ABTS radical stock solution to 80 volumes of 20 mM PB (pH 6.8) and adding, if necessary, PB or ABTS stock solution to adjust the absorbance to about 0.83 at 734 nm. The ABTS radical stock solution contains 2.5 mM K₂S₂O₈ and 6.9 mM ABTS.

### Quenching of Alkyl Peroxyl Radicals

Analyses may be performed to determine the antioxidant activity of peptides by measuring the quenching of alkyl peroxyl radicals. A t-butoxyl peroxide/beta-carotene assay was used based on several published assays (Akaike, et al., J. Agric. Food Chem. 43:1864 (1995); Nakao et al., Phytochem.49:2379 (1998); Burton et al., Science 224:569 (1984)). This assay involves two sequential reactions:
(i) t-BuOOH + MetMb ? ≡t-BuOO.
(ii) t-BuOO· + beta-carotene ? bleaching

The reaction of the peroxyl radical with beta-carotene results in the disappearance of yellow color associated with beta-carotene (*i*.*e*., bleaching). Absorbance measured at 452 nm indicates that antioxidants effectively compete for peroxyl radical and prevent bleaching. Results from this test indicate the relative alkyl peroxyl radical quenching capacity of peptides compared to beta-carotene.

The assay is performed by combining 725 µl of 20 mM phosphate buffer (PB, pH 7.4) with 120 µl of Tween 20 (2.4%) and 120 µl of MetMb (50 µM MetMb made from mixing 1:1 v/v of 100 µM myoglobin (Sigma, St. Louis, MO) and 100 µM potassium ferricyanide (Aldrich, Milwaukee, WI) in PB). The mixture is incubated at 37°C. Beta-carotene (saturated solution in acetone at 37°C) is added and the solution is mixed. Then, 25 µl of peptides or Trolox standard (0.5 to 10 mM) and 150 µl of 50 mM t-BuOOH in PB are added. The sample is mixed and absorbance is read at 452 nM at 30s and then one minute intervals for about 10 or 12 minutes. 50% acetonitrile is used as a blank where appropriate.

### FOX Assay of Linoleic Acid Oxidation

Unsaturated fatty acid is liable to oxidation. This may be due to direct oxidation by molecular oxygen or facilitated by transition metal ions or other radicals. The initial oxidized products are peroxides. This assay was developed to evaluate the activity of antioxidants, including peptides, in preventing the oxidation of linoleic acid. The concentration of peroxides generated is measured according to the methods of Jiang, et al. (Analyt. Biochem. 202:384 (1992)) and Lowum, et al. (J. Food Biochem. 13:391 (1989)). Standard curves are prepared using hydrogen peroxide at a series of known concentrations. The assay is performed as follows:
1. Emulsion: make two 120 µl Tween 20, 1 ml linoleic acid of PB (20 mM, pH 7.4) or citric acid-phosphate buffer (0.2125 M Na₂HPO₄mixed with 0.1 citric acid to pH 4.1 and diluted 30 ml to 200 ml) to total 32 ml in 50 ml capped centrifuge tubes; vortex about five minutes to form a white, milk-like emulsion and mix the two to have the same emulsion for triplicates of six or seven samples.
2. Sample: 1.6 ml dH₂O and 0.4 ml 50% ACN as control or 2.5 mM or 5 mM sample in 50% ACN in 12 ml capped centrifuged tubes.
3. Take 8 ml of emulsion and mix with the 2 ml control or sample in the 12 ml capped centrifuged tube; vortex two minutes; take 3 ml into a 20 ml open scintillation vial with a stirring bar; make triplicates of each sample. The rest (about 1 ml) is used for zero time point of each sample and mixed with FOX reagent as step 5.
4. Incubate and stir at 37°C in open air.
5. At determined time points (usually 30 minute intervals), aliquot 50 µl of the reaction emulsion and mix with 950 µl FOX reagent in an Ep-tube and vortex.
6. Allow to stand for over 30 minutes (40 minutes-1 hour), read at A560 nm (dH₂O as blank).

FOX reagent is prepared by combining 900 ml MeOH, 100 ml 250 mM H₂SO₄, 880 mg BHT, 76 mg Xylenol Orange and 98 mg ammonium sulfate hexahydrate. A standard curve is prepared by diluting hydrogen peroxide to get 25, 50, 100, 250, 500, 600, 800, and 1000 µM concentrations with appropriate buffer and 50 µl solution is mixed with 950 µl FOX reagent. Readings are taken at 1 hour at A560 nm. The final Trolox concentration in the reaction is 0.1 mM. The final concentration of the samples (Genistein or peptides) is 0.2 mM.

### Quenching of Superoxide Ion

This analysis is performed to evaluate the ability of peptides to quench the superoxide ion. The superoxide ion assay is performed according to the methods of Yen, et al. (J. Agric. Food Chem. 43:27 (1995) and Nishikimi, et al. (Biochem. Biophys. Res. Comm. 46:849 (1972)). For this assay, 830 µl PB (0.1 M, pH 7.4), 120 µl 0.15 mM phenazine methosulfate (PMS) in 0.1 M phosphate buffer, pH 7.4, 120 µl 0.375 mM nitro blue tetrazolium (NBT) in phosphate buffer and 50 µl sample or sample solvent are combined in Ep-tube. To this solution, 100 µl 2.34 mM NADH solution is added to start the reaction. The solution is agitated and absorbance is read at 560 nm after five minutes. Sample solvent is used as a control.

## Claims

1. A method of reducing the oxidative degradation of fats or oils in a composition, comprising adding to said composition an effective amount of an isolated protein consisting of any of the following sequences:
GVAW;
GVAWW;
GVAWWM;
GVAWWMY;
GVAWWM*; and
GVAWWM*Y, wherein M* is methionine sulfoxide.

## Patentansprüche

1. Verfahren der Reduzierung des oxidativen Abbaus von Fetten oder Ölen in einer Zusammensetzung, bei dem man der Zusammensetzung eine wirksame Menge eines isolierten Proteins zusetzt, das aus einer der folgenden Sequenzen besteht:
GVAW,
GVAWW,
GVAWWM,
GVAWWMY,
GVAWWM* und
GVAWWM*Y, worin M* Methioninsulfoxid ist.

## Revendications

1. Procédé pour réduire la dégradation oxydative des matières grasses ou des huiles dans une composition, comprenant l'addition à ladite composition d'une quantité efficace d'une protéine isolée consistant en l'une quelconque des séquences suivantes :
GVAW ;
GVAWW ;
GVAWWM ;
GVAWWMY ;
GVAWWM* ; et
GVAWWM*Y, dans laquelle M* est l'acide sulfonique de méthionine.
